# EUROPEAN PATENT APPLICATION

(11) **EP 2 135 555 A1**
(43) Date of publication of application: **23.12.2009**
(21) Application number: 09162556.6
(22) Date of filing: 12.06.2009
(51) Int. Cl.: A61B 8/00, G01S 15/89

(54) **Operation control of an ultrasound system based on an impact pattern applied thereto**

(30) Priority: 18.06.2008 KR 20080057397
(71) Applicant: MEDISON CO., LTD., Kangwon-do 250-870 (KR)
(72) Inventor: Song, Young Seuk, 1003 Daechi-dong, Gangnam-gu 135-851, Seoul (KR); Park, Jong Min, 1003 Daechi-dong, Gangnam-gu 135-851, Seoul (KR); Lee, Jin Yong, 1003 Daechi-dong, Gangnam-gu 135-851, Seoul (KR)
(74) Representative: Schmid, Wolfgang

(57) **Abstract**

There are disclosed embodiments for controlling an operation of an ultrasound system based on an impact applied thereto. In the ultrasound system designed to operate in a plurality of diagnostic modes, a user may be verified based on the impact pattern applied to the ultrasound system. Also, a desirable diagnostic mode of the ultrasound system may be selected and executed based on the impact pattern. The ultrasound system may include an ultrasound diagnostic unit to form ultrasound images based on ultrasound echo signals reflected from a target object. An impact detecting unit may detect impacts applied to the ultrasound diagnostic unit from a user to thereby output impact pattern information. A control unit may control an operation of the ultrasound diagnostic unit based on the impact pattern information so that the diagnostic mode corresponding to the impact pattern information may be executed.

## Description

The present application claims priority from Korean Patent Application No. 10-2008-0057397 filed on June 18, 2008, the entire subject matter of which is incorporated herein by reference.

### TECHNICAL FIELD

The present disclosure relates to an ultrasound system, and more particularly to an ultrasound system configured to be controlled based on impact patterns applied by a user.

### BACKGROUND

Recently, an ultrasound system has been extensively used in the medical field due to its non-invasive and non-destructive nature. The ultrasound system operates in various image modes such as a brightness mode, a Doppler mode and the like to acquire ultrasound images for diagnosis. Also, the ultrasound system may operate in a measure mode for measuring volumes of parts of interest (e.g., organs, lesions, etc.) contained in a target object.

Since the ultrasound system is relatively an expensive equipment and private medical information may be recorded therein, the security system may be set in the ultrasound system to prevent any unauthorized access thereto. The security system of the ultrasound system may ask a user to input identification and password. The user may operate the ultrasound system only after the identification and password are verified. The input of the user's identification and password may be implemented through a user interface such as a control panel, a touch screen or the like.

The ultrasound system typically operates in various diagnostic modes such as 2-dimensional mode, color Doppler mode, power Doppler mode, color flow mode and the like. The diagnostic modes may include a single mode for executing a single diagnostic mode and a complex mode for executing at least two or more diagnostic modes at the same time. Also, the diagnostic modes may further include a 3-dimensional mode for forming a 3-dimensional image. The diagnostic mode may be selected by a user through a user interface such as a control panel, a touch screen or the like. In such a case, the user has to manipulate the control panel or the touch screen to input a selection of the diagnostic mode while holding a probe with one hand. Thus, the user tends to inevitably examine a patient at an uncomfortable posture.

### SUMMARY

An embodiment for operating an ultrasound system in response to an impact applied by a user is disclosed herein. In one embodiment, by way of non-limiting example, an ultrasound system designed to operate in a plurality of diagnostic modes, comprises: an impact detecting unit operable to detect impacts applied to the ultrasound system by a user to thereby output impact pattern information; and a control unit operable to control an operation of the ultrasound system based on the impact pattern information.

In another embodiment, a method of operating an ultrasound system, wherein the ultrasound system operates in a plurality of diagnostic modes and includes a storage unit, an ultrasound diagnostic unit, an impact detecting unit and a control unit, comprises: a) storing impact pattern information associated with user information and a mapping table mapping impact pattern information with the diagnostic modes in the storage unit; b) at the impact detecting unit, detecting an impact applied to the ultrasound diagnostic unit from a user to thereby output first impact pattern information; c) at the control unit, retrieving the impact pattern information associated with the user information from the storage unit to identify a user corresponding to the first impact pattern information, and controlling the operation of the ultrasound diagnostic unit based on the identified user; d) at the impact detecting unit, detecting an impact applied to the ultrasound diagnostic unit from a user to thereby output second impact pattern information; and e) at the control unit, retrieving the impact pattern information of the mapping table from the storage unit to identify a diagnostic mode corresponding to the second impact pattern information, and controlling the operation of the ultrasound diagnostic to execute the identified diagnostic mode.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

- FIG. 1: is a block diagram showing an illustrative embodiment of an ultrasound system.
- FIG. 2: is a schematic diagram showing an example of impact pattern information to be used to identify a user.
- FIG. 3: is a schematic diagram showing an example of a mapping table for mapping impact pattern information into diagnostic modes.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

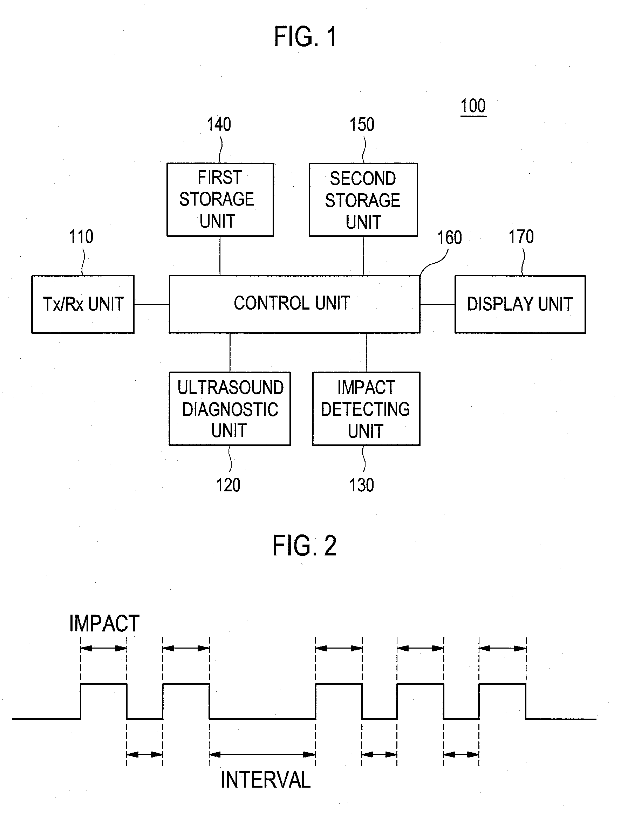

FIG. 1 is a block diagram showing an illustrative embodiment of an ultrasound system. As depicted in FIG. 1, the ultrasound system 100 may include a transmission/reception (Tx/Rx) unit 110 that may be operable to transmit an ultrasound transmit beam to a target object. The ultrasound transmit beam may be formed with a plurality of ultrasound signals. The Tx/Rx unit 110 may be further operable to receive ultrasound echoes reflected from the target object to thereby output receive signals. In one embodiment, the Tx/Rx unit 100 may include a probe (not denoted) containing a plurality of elements for reciprocally converting electrical signals and ultrasound signals.

The ultrasound system 100 may further include an ultrasound diagnostic unit 120. The ultrasound diagnostic unit 120 may be operable to form ultrasound images based on the receive signals outputted from the Tx/Rx unit 110. In one embodiment, the ultrasound diagnostic unit 120 may include a beam former (not shown), a signal processor (not shown) and an ultrasound image processor (not shown). Although the signal processor and ultrasound image processor are described as separate elements in one embodiment, the signal processor and the ultrasound image processor may be embodied with a single processor. The beam former may be operable to perform transmit-focusing to form an ultrasound transmit beam. The beam former may be further operable to perform receive-focusing upon the receive signals to form a receive beam. The signal processor may perform signal processing such as amplification, gain adjustment and the like upon the receive beam. The ultrasound image processor may be operable to form ultrasound images based on the signal-processed receive beam.

The ultrasound system 100 may further include an impact detecting unit 130. In one embodiment, the impact detecting unit 130 may be operable to detect impacts applied to the ultrasound diagnostic unit 120 by a user to thereby output impact pattern information. The pattern information may include the number of applied impacts, amplitude of sound generated by the impact, intervals between the impacts and the like. In one embodiment, any type of detecting units capable of detecting the impacts applied by a user may be adopted as the impact detecting unit 130. For example, the detecting unit 130 may include an impact sensor, a piezoelectric sensor, an acoustic sensor or the like. In one embodiment, the impact detecting unit 130 may be embodied with a foot switch.

The ultrasound system 100 may further include a first storage unit 140. The storage unit 140 may store impact pattern information associated with user information. As shown in FIG. 2, the impact pattern information may be represented with intervals between the impacts, e.g., long and short intervals between the impacts, but is not limited thereto. In one embodiment, the impact pattern information represented by the intervals between the impacts may be used to identify a user.

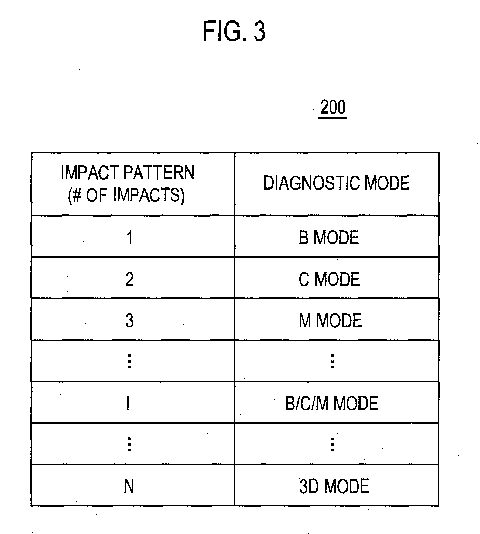

The ultrasound system 100 may further include a second storage unit 150. The second storage unit 150 may store a mapping table in which the impact pattern information is associated with respective diagnostic modes. In one embodiment, the second storage unit 150 may store the mapping table, in which the numbers of applied impacts are associated with the diagnostic modes, as shown in FIG. 3. Although it is described that the impact pattern information is represented by the numbers of impacts, the impact pattern information is certainly not limited thereto.

The ultrasound system 100 may further include a control unit 160. The control unit 160 may be operable to retrieve the impact pattern information stored in the first storage unit 140 to identify a user corresponding to the impact pattern information outputted when the ultrasound diagnostic unit 120 starts its operation. The control unit 160 may be further operable to control an operation of the ultrasound diagnostic unit 120 based on the identified user. In one embodiment, if the impact pattern information receives from the impact detecting unit 130, then the control unit 160 may be operable to search the first storage unit 140 to check whether the impact pattern information is stored in first storage unit 140, which matched with the received impact pattern information. If the impact pattern information, which coincides with the inputted impact pattern information, exists in the first storage unit 140, then the control unit 160 may be operable to identify a user corresponding to the impact pattern information and control an operation of the ultrasound diagnostic unit 120 based on the identified user. That is, the ultrasound diagnostic unit 120 may operate in response to the impacts of the user without any input through the control panel or the touch screen.

On the contrary, if the impact pattern information, which coincides with the inputted impact pattern information, does not exist in the first storage unit 140, then the control unit 160 may be operable to output a message for requesting an input of user identification again.

If the impact pattern information receives from the impact detecting unit 130 while the ultrasound diagnostic unit 120 operates, then the control unit 160 may be further operable to control the diagnostic mode of the ultrasound diagnostic unit 120 in response to the inputted impact pattern information. In one embodiment, if the impact pattern information is received, then the control unit 160 may be operable to search the second storage unit 150 to check whether the impact pattern, which coincides with the inputted impact pattern information, is stored in the second storage unit 150. If the impact pattern information, which coincides with the inputted impact pattern information, exist in the second storage unit 150, then the control unit 160 may be operable to control an operation of the ultrasound diagnostic unit 120 such that the diagnostic mode corresponding to the inputted impact pattern information is executed therein. For example, if the impact pattern information may represent a once impact, then the control unit 160 may be operable to control an operation of the ultrasound diagnostic unit 120 such that a B-mode is executed. Also, if the impact pattern information may represent a twice impact, then the control unit 160 may be operable to control an operation of the ultrasound diagnostic unit 120 such that a C-mode is executed.

In one embodiment, a tolerable time between successive impacts to be recognized as one instruction may set to prevent an undesirable execution of the diagnostic mode. For example, if a time interval between successive impacts is within the tolerable time, then the impacts may be recognized as one instruction. On the contrary, if a time interval between successive impacts is beyond the tolerable time, then the impacts may not be recognized as one instruction.

In one embodiment, the control unit 160 may be operable to control operations of other elements. The ultrasound system 100 may further include a display unit 170 that may display the ultrasound images formed in the ultrasound diagnostic unit 120. Also, the display unit 170 may display the message outputted from the control unit 160, which may be displayed with texts. In one embodiment, it is described that the message outputted from the control unit 160 is displayed on the display unit 170 with texts, but the message may not be limited thereto. In another embodiment, the message may be outputted with sounds.

In one embodiment, although it is described that the impact detecting unit 130, the first storage unit 140, the second storage unit 150 and the control unit 160 are separately configured from the ultrasound diagnostic unit 120, the impact detecting unit 130, the first storage unit 140, the second storage unit 150 and the control unit 160 may be fabricated in the ultrasound diagnostic unit 120 in another embodiment.

As mentioned above, since the ultrasound diagnostic unit 120 is operated in response to the impact of the user, the user may be easily identified without any complex identification procedure and the diagnostic mode may be also easily changed.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system for operating a plurality of diagnostic modes, comprising:
an impact detecting unit operable to detect impacts applied to the ultrasound system by a user to thereby output impact pattern information; and
a control unit operable to control an operation of the ultrasound system based on the impact pattern information.

2. The ultrasound system of Claim 1, further comprising an ultrasound diagnostic unit operable to form ultrasound images based on ultrasound echo signals reflected from a target object, wherein the impacts are applied to the ultrasound diagnostic unit.

3. The ultrasound system of Claim 2, further comprising a storage unit configured to store impact pattern information associated with user information and a mapping table mapping previously set impact pattern information with the diagnostic modes.

4. The ultrasound system of Claim 3, wherein the control unit is configured to reference the storage unit to retrieve the impact pattern information stored in the storage unit to identify a user corresponding to the impact pattern information outputted from the impact detecting unit when starting to operate the ultrasound diagnostic unit.

5. The ultrasound system of Claim 4, wherein the control unit is further configured to check impact pattern information of the mapping table stored in the storage unit to identify a diagnostic mode corresponding to the impact pattern information outputted from the detecting unit, the control unit being further operable to control an operation of the ultrasound diagnostic unit based on the identified diagnostic mode.

6. The ultrasound system of Claim 5, wherein the impact pattern information include at least one of the number of the impacts, intervals of the impacts and amplitude of a sound generated by the impacts.

7. The ultrasound system of Claim 5, further comprising an output unit configured to output the identification result with display or sound under the control of the control unit.

8. The ultrasound system of Claim 7, wherein the impact detecting unit may include at least one of an impact sensor, an acoustic sensor and a piezoelectric sensor.

9. A method of operating an ultrasound system, wherein the ultrasound system operates in a plurality of diagnostic modes and includes a storage unit, an ultrasound diagnostic unit, an impact detecting unit and a control unit, the method comprising:
a) storing impact pattern information associated with user information and a mapping table mapping impact pattern information with the diagnostic modes in the storage unit;
b) at the impact detecting unit, detecting an impact applied to the ultrasound diagnostic unit by a user to thereby output first impact pattern information;
c) at the control unit, retrieving the impact pattern information associated with the user information from the storage unit to identify a user corresponding to the first impact pattern information, and controlling the operation of the ultrasound diagnostic unit based on the identified user;
d) at the impact detecting unit, detecting an impact applied to the ultrasound diagnostic unit by a user to thereby output second impact pattern information; and
e) at the control unit, retrieving the impact pattern information of the mapping table from the storage unit to identify a diagnostic mode corresponding to the second impact pattern information, and controlling the operation of the ultrasound diagnostic to execute the identified diagnostic mode.

10. The method of Claim 9, wherein the first and second impact pattern information include at least one of the number of the impacts, time duration of impact intervals and amplitude of a sound generated by the impacts.

11. The method of Claim 10, wherein the ultrasound system further includes an output unit, the method further comprising outputting the identification result with display or sound under the control of the control unit.

12. The method of Claim 9, wherein the impact detecting unit may include at least one of an impact sensor, an acoustic sensor and a piezoelectric sensor.
